# EUROPEAN PATENT APPLICATION

(11) **EP 3 225 150 A1**
(43) Date of publication of application: **04.10.2017**
(21) Application number: 17161143.7
(22) Date of filing: 15.03.2017
(51) Int. Cl.: A61B 1/00, A61M 16/08

(54) **CONNECTOR AND TUBE SET FOR USE IN A MEDICAL PROECEDURE**

(30) Priority: 31.03.2016 GB 201605430
(71) Applicant: KeyMed (Medical & Industrial Equipment) Ltd, Southend-on-Sea Essex SS2 5QH (GB)
(72) Inventor: ROBERTS, Tim, Southend-on-Sea, Essex (GB)
(74) Representative: Boult Wade Tennant

(57) **Abstract**

A connector (10) for connecting a medical tube set to a medical container, comprises a collar (12) connectable to a container, a cap (14) comprising at least one opening (30) for receiving a tube of a tube set, and an annular seal (16). The cap (14) is located in the collar (12) between an inner surface of the collar and the seal (16). The cap (14) is rotatable relative to the collar (12). The collar (12) comprises a generally cylindrical side wall open at its upper and lower ends and an annular flange projects inwardly from the side wall at the upper end. The cap (14) comprises a disc defining at least one opening and having an outer annular rim which locates between a lower surface of the annular flange on the collar (12) and the seal (16).

## Description

The present invention relates to a connector for connecting a medical tube set to a medical container such as a water bottle for use in a medical procedure such as an endoscopy. Specifically, the invention relates to a connector with cap for receiving the tubes of a tube set, which is rotatable relative to other parts of the connector, and to a tube set incorporating such a connector.

When carrying out an endoscopy, it is usual to provide a supply of liquid, such as water, and a supply of gas, such as CO₂, to an endoscope. Water or other liquid may be used to clean the lens at the distal end of the endoscope and to irrigate or flush the site being viewed to clean away debris. Gas may be supplied to insufflate the body cavity under inspection. In many existing systems, two separate water bottles are provided, one bottle providing water for lens cleaning and the other providing water for flushing. In each case, a tube for carrying the water may pass through a cap fitted to the water bottle and have its free end below the surface of the water. The other end of the tube will be connected to the endoscope. Insufflation gas may be supplied through a second tube into one bottle to fill the space above water. A third tube may then be provided to take the insufflation gas from the bottle to the endoscope.

Thus, users need to prepare two water bottles, with replacements/refills available as necessary, and the tube sets must be connected to each bottle as required. In addition, different bottle caps are required to suit different tube sets. This increases the complexity of the system and the set-up time required and may also delay the procedure if bottles need to be changed partway through.

The present invention provides a connector for connecting a medical tube set to a medical container, comprising a collar connectable to a container, a cap comprising at least one opening for receiving a tube of a tube set, and an annular seal, wherein the cap is locatable in the collar between an inner surface of the collar and the seal, and wherein the cap is rotatable relative to the collar, wherein the collar comprises a generally cylindrical side wall open at its upper and lower ends, with an annular flange projecting inwardly from the side wall at the upper end, and the cap comprises a disc defining at least one opening and having an outer annular rim, wherein the annular rim locates between a lower surface of the annular flange on the collar and the seal.

In this way, as the collar is connected to or disconnected from a container, it can rotate while the cap remains stationary. This avoids twisting or straining tubes connected through the cap.

The seal may contact an inner surface of the collar and a surface of the cap.

Preferably, cylindrical tube receiving portions project from the cap around the or each opening.

The cap may comprise four openings, and the openings may comprise two or more different diameters.

The present invention also provides a tube set comprising a connector as described above, wherein the cap comprises four openings and a tube is fitted through each opening.

In this way, a hybrid tube set is provided which can be used to suit different applications, as required,

In a preferred embodiment, first and second tubes are adapted to supply liquid from a container to a medical instrument. A third tube may be adapted to pass gas between a container and a medical instrument. A fourth tube may be adapted to supply gas from an external source into a container.

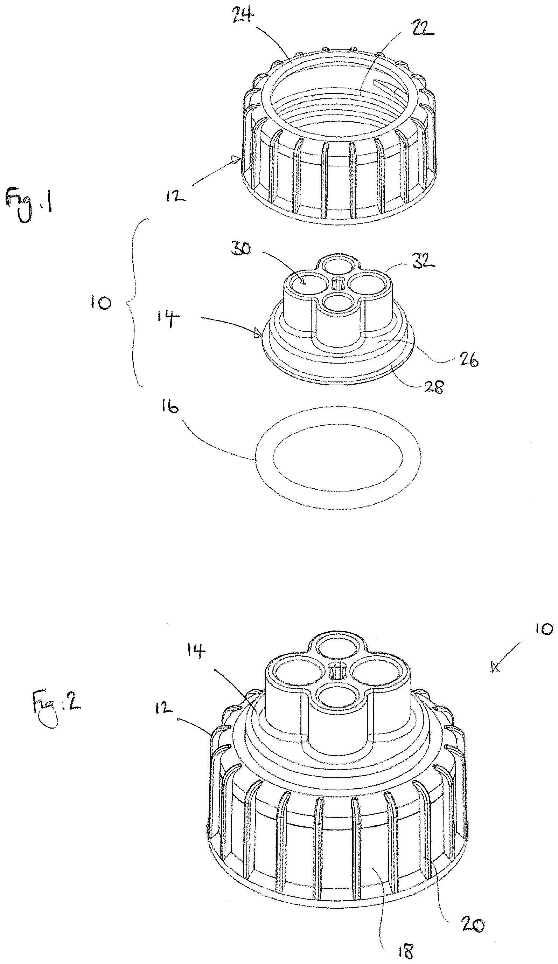

The invention will now be described in detail, by way of example only, with reference to the accompanying drawings, in which:-
Figure 1 is an exploded view of the components forming a connector in accordance with embodiment of the present invention;
Figure 2 is perspective view of the assembled connector;
Figure 3 shows a top of the assembled connector of Figure 2;
Figure 4 is cross-section of Figure 3 along the line AA;
Figure 5 is a front view of a tube set incorporating the connector;
Figure 6 is a front view of the tube set of Figure 5; and
Figure 7 is a side view of part of the tube set of Figures 5 and 6 connected to a bottle.

A connector 10 in accordance with the presence invention comprises an annular collar 12, a cap 14 for receiving tubes of a tubing set and a seal 16.

The collar 12 comprises an annular side wall 18 open at its upper and lower ends. The exterior of the collar 12 may be provided with ribs 20 or other surface formations to assist with a user gripping a collar in use. The interior of the collar 12 is provided with means for connection to a container, such as a thread 22 for screwing onto the neck of a water bottle. The thread 22 is preferably formed with a relatively large pitch and relatively deep threads so that it is capable of fitting on a number of different bottle or container necks which have different dimensions. An annular flange 24 projects from the side wall 18 inwardly around the open upper end of the collar 12.

The cap 14 consists of a circular base 26 with an outer annular rim 28. A number of openings 30 are provided through the base 26 for receiving tubes of a tube set. In this example, the cap 14 is provided with four openings each surrounded by tube receiving portions 32 which projects upwardly from the base 26. These may be provided in different diameters to suit different tubes. In this case, there are two larger diameter openings and two smaller diameter openings. The tube receiving portions 32 support and protect the tubes as they pass through the cap 14.

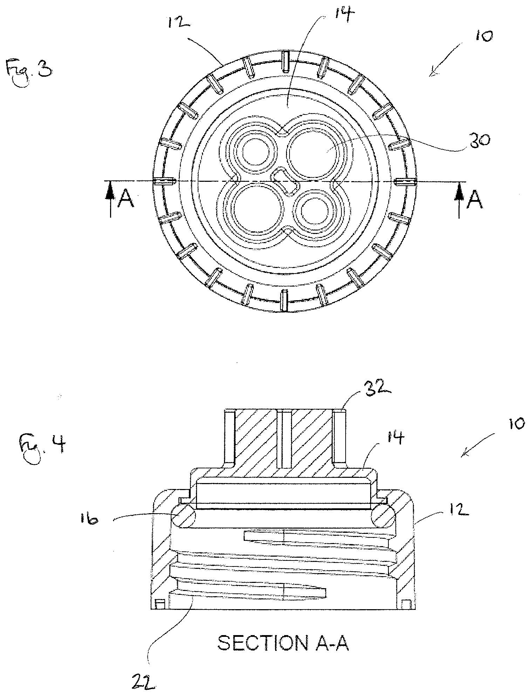

When assembled, the rim 28 of the cap 14 is received between the flange 24 on the collar 12 and the seal 16, as best seen in Figure 4. The cap 14 is able to rotate relative to the collar 12. Thus, when the collar 12 is screwed onto a bottle neck, or unscrewed therefrom, it can rotate while the cap 14 remains stationary, so that the tubes connected through the openings 30 and the tube receiving portions 32 are not twisted.

In use, the tubes of a required tube set are fitted through the openings 30 and cylindrical tube receiving portions 32. The connector 10 is attached to the neck of a water bottle or similar container. As the collar 12 is screwed onto the bottle neck, it is able to rotate relative to the cap 14, which can remain stationary. This avoids twisting or putting strain on the tubes of the tube set. Similarly, if the bottle is empty and needs replacing during a procedure, or at the end of the procedure, the collar 12 can be unscrewed from the bottle neck without causing rotation of the cap 14 and twisting or straining of the tubes fitted through the cap 14.

As mentioned above, the cap 14 preferably has four tube receiving portions 30 of varying diameter. These can accommodate, for example, a tube supplying gas into the bottle, a tube for passing gas from the bottle to the endoscope, a tube for passing water from the bottle to the endoscope for lens cleaning, and a tube for passing water to the endoscope for irrigation of the site under inspection. If fewer than four tubes are needed and not all of the openings 30 and tube receiving portions 32 are required, they may be left open, for example, to allow air into the bottle, or the unused tube receiving portions 32 may be closed with a suitable stopper or bung.

In this way, the connector 10 can be used in a variety of different procedures where different forms of tube set are required and a hospital or medical facility need only stock one form of connector which is adaptable to different applications.

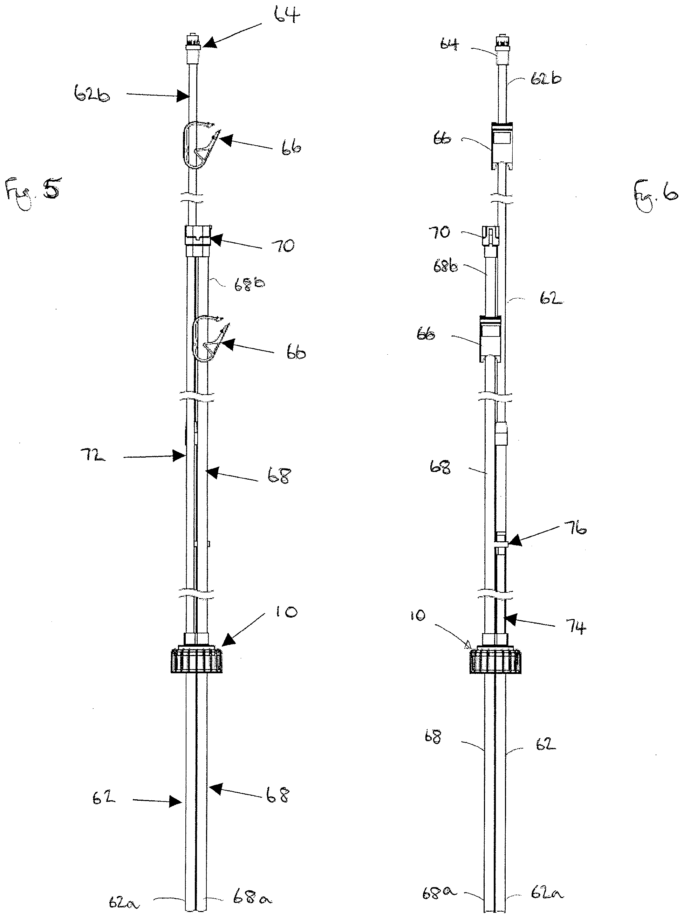

Figures 5 and 6 illustrate a tube set 60 which incorporates the connector 10 and has four tubes 62, 68, 72, 74 fitted through the cap 14.

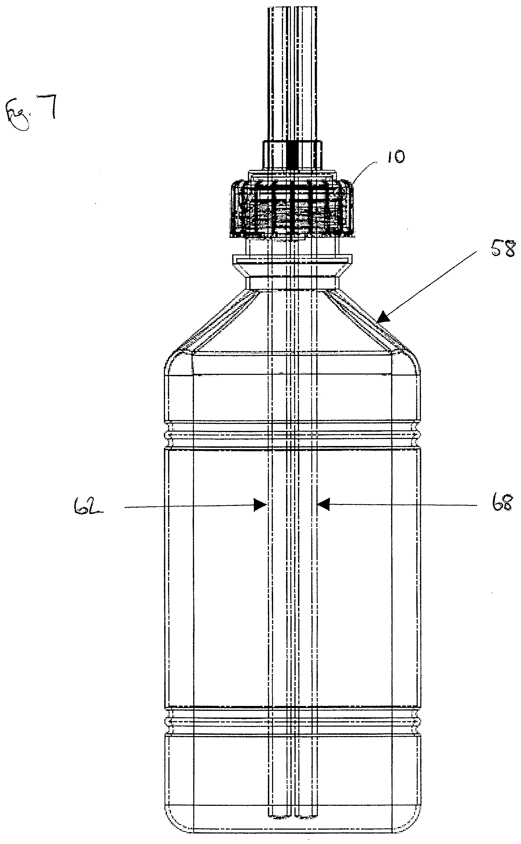

Figure 7 shows the connector 10 and parts of the tubes when connected to a typical water bottle 58.

In this example, the first tube 62 comprises an irrigation tube 62 which is used to pass water (or other liquid) from a bottle to which the connector 10 is connected to an endoscope, to irrigate a site under observation. The proximal end 62a of the irrigation tube 62 passes through the connector 10 and is dimensioned to extend well into a bottle so that it will be close to the bottom surface of the bottle when the connector 10 is secured to the neck. The distal end 62b of the irrigation tube 62 includes a connector 64 for connecting the irrigation tube 62 to an endoscope. Typically, the connector 64 will include a one-way valve to prevent backflow of liquid from the endoscope into the tube 62. Usually, the irrigation tube 62 will be provided with a conventional clamp 66. This is illustrated in the open position, but in the closed position, this squeezes the tube 62 to close it, to prevent leakage when it is disconnected from an endoscope.

The second tube 68 comprises a rinsing tube 68 which is used to pass water (or other liquid) from a bottle to which the connector 10 is connected to an endoscope to rinse the lens and prevent the view being obscured. As with the irrigation tube 62, the rinsing tube 68 includes a proximal end 68a extending well into the bottle so that in use it will be close to the bottom surface. The distal end 68b includes a connector 70 for connection to an endoscope. As described below, the connector 70 may be common with a connector at the end of a third tube 72. The rinsing tube 68 may also be fitted with a clamp 66 to close off the tube 68 when it is disconnected from an endoscope to prevent leakage.

The third tube is a gas tube 72 (also commonly referred to as an air tube) which is used to supply air (or other gas) from a bottle to which the connector 10 is connected to an endoscope. The proximal end 72a terminates just below the seal 14 in the connector 10 so that it is well above the level of water in the container. The distal end 72b includes a connector for connection to the endoscope and, as noted above and illustrated in Figures 8 and 9, this may be combined with the connector 70 for the rinsing tube 68.

A fourth tube 74 is a gas supply tube, for example, for supply CO₂ into the bottle. The proximal end 74a terminates just beneath the seal 14 and well above the level of any water in the container. The distal end 74b has a connector 76 including a check valve, for connection to an external gas supply.

In use, the tube set 60 is connected to a bottle or other container by screwing the connector 10 onto the bottle neck. The irrigation and rinsing tubes 62, 68 extend into the liquid within the bottle. If an external gas supply is used, gas is passed into the bottle to fill the space above the water. The gas pressurises the bottle to help water flow through the irrigation and rinsing tubes 62, 68. Gas may also be passed from the bottle through the gas tube 72 to the endoscope for insufflation purposes.

The tube set 60 can also be used without the external gas supply. In this case, the gas supply tube 74 is not used and is closed by virtue of the check valve in the connector at its distal end 74b. In this case, compressed air is normally supplied separately to the endoscope and the endoscope includes a control button which, when pressed by a user directs air from the separate supply for the endoscope back into the bottle via the gas tube 72. This pressurises the bottle to force water through rinsing tube 68.

In this way, the tube set 60 provides a hybrid system which can be used with a single water bottle to supply both irrigation and rinsing liquid to an endoscope and it can be used either with a gas supply from an external source for insufflation or can be used to utilise air otherwise supplied to the endoscope.

A hospital may stock the assembled hybrid tube set 60 as described above which can be used as desired, making use of all four tubes or with unused tubes closed by the clamps 66 or by virtue of the check valves in the relevant connectors.

The components of the connector 10 and tube set 60 may be formed as simple plastic mouldings. They may be intended for single use and subsequent disposal, or they may be formed from materials which are suitable for sterilisation and re-use.

## Claims

1. A connector for connecting a medical tube set to a medical container, comprising a collar connectable to a container, a cap comprising at least one opening for receiving a tube of a tube set, and an annular seal, wherein the cap is located in the collar between an inner surface of the collar and the seal, and the cap is rotatable relative to the collar wherein the collar comprises a generally cylindrical side wall open at its upper and lower ends, and an annular flange projects inwardly from the side wall at the upper end, and the cap comprises a disc defining at least one opening and having an outer annular rim, wherein the annular rim locates between a lower surface of the annular flange on the collar and the seal.

2. A connector is claimed in claim 1, wherein the seal contacts an inner surface of the collar and a surface of the cap.

3. A connector as claimed in any preceding claim, wherein cylindrical tube receiving portions project from the cap around the or each opening.

4. A connector as claimed in any preceding claim, wherein the cap comprises four openings.

5. A connector as claimed in claim 4, wherein the openings comprise two or more different diameters.

6. A tube set comprising a connector as claimed in claim 1, wherein the cap comprises four openings and a tube is fitted through each opening.

7. A tube set as claimed in claim 6, wherein first and second tubes are adapted to supply liquid from a container to which the connector is connected to a medical instrument.

8. A tube set as claimed in claim 6 or claim 7, wherein a third tube is adapted to pass gas between the container to which the connector is connected and a medical instrument.

9. A tube set as claimed in any of claims 6-8, wherein a fourth tube is adapted to supply gas from an external source to a container to which the connector is connected.
